Europäisches Patentamt

European Patent Office

Office européen des brevets

(19)

(11) Publication number : **0 459 613 A1**

# (12) EUROPEAN PATENT APPLICATION

(21) Application number : 91303314.8

(22) Date of filing : 15.04.91

(51) Int. Cl.$^5$ : **G01N 27/00, G01N 29/00**

(30) Priority : 01.06.90 GB 9012247

(43) Date of publication of application :
04.12.91 Bulletin 91/49

(84) Designated Contracting States :
DE FR IT NL

(71) Applicant : GEC-MARCONI LIMITED
The Grove, Warren Lane
Stanmore, Middlesex HA7 4LY (GB)

(72) Inventor : Stevenson, Adrian Carl
15 Stoke Road
Blisworth Northamptonshire NN7 3BZ (GB)
Inventor : Brierley, Crofton John
Estria Fosters Booth Road
Pattishall Northamptonshire NN12 8JU (GB)
Inventor : Paige, Edward George Sydney
6, Mill Lane
Horton-cum-Studley Oxford (GB)

(74) Representative : Pope, Michael Bertram
Wingate
Central Patent Department Wembley Office
The General Electric Company, p.l.c. Hirst
Research Centre East Lane
Wembley Middlesex HA9 7PP (GB)

(54) Biosensors.

(57) A liquid phase biosensing device, using surface skimming bulk waves (SSBW), is described in which electrodes (12) are formed on the surface of a piezoelectric substrate (10). The electrodes (12) are photolithographically deposited and are elongate. A biosensitive protein is immobilised on the surface of the device.

EP 0 459 613 A1

DEFORMED PROTEIN LAYER

QUARTZ CRYSTAL

Fig. 1.

This invention relates to biosensors and to devices incorporating such sensors for detecting, for example, antibody-antigen binding reactions. Antibodies are molecules which protect an organism from disease and/or infection. When an organism is exposed to an antigen, the organism creates antibodies which provide binding sites whereto to bind and immobilise the antigen.

Many known antibodies exist and can be used to detect specific antigens. Detection is effected of such antigens by providing the appropriate antibody and detemining whether such binding takes place thus indicating the presence (or absence) of the antigen. For example, an antibody may be provided on a surface of a support, the support may be immersed in a liquid thought to contain an antigen, and a physical characteristic of the surface and/or the support monitored to determine if binding (indicative of the presence of the antigen) has taken place. In such testing, the support may be a piezo-electric crystal. Surface Acoustic Waves (SAW) have been used to detect an antigen, by monitoring attenuation of the waves caused by binding of the antigen to the antibody on the surface of the support.

A paper describing such work is "The potential of the Bulk Acoustic Wave Device as a Liquid-Phase Immunosensor" by Michael Thompson et al; IEEE Transactions on Ultrasonics, Ferroelectrics, and Frequency Control,; Volume UFFC-34, No. 2, March 1987; at pages 127-135.

A problem with the devices described in the aforesaid paper is that considerable penetration of the environment to be sensed by the acoustical wave, occurs. If the environment is gaseous, the problem is not unduly onerous. The preferred environment for such tests is a liquid environment and the penetration causes considerable damping and thereby reduces the sensitivity of the device.

Work has been effected by other researchers who have considered Rayleigh Surface Acoustic Waves (SAW's), Bleustein-Gulyaev (BG) waves and Surface Skimming Buk Waves (SSBW's), their means of propagation and their attenuation in liquid phase environments. Two publications by Fabien Josse and Zack Shana entitled "Analysis of shear horizontal surface waves at the boundary between a piezoelectric crystal and a various Muid medium." (Journal Acoustic Society of America 84(3), September 1988, Pgs. 978-984) and "Effect of liquid relaxation time on SH surface waves liquid sensors" (Journal Accoustic Society of America 85(4), April 1989, Pgs. 1566- 1569) and a publication by Masatsune Yamaguchi, Ken-ya Hashimoto, Yasuharu Nakajima and Hiroshi Kogo entitled "Surface Skimming Bulk Wave and Bleustein-Gulyaev Wave on Rotated Y-cuts of Quartz" published by the Department of Electrical Engineering, Chiba University, Yayoi-cho, Chiba-shi, Japan 260 (some parts of which were republished in "Electronics Letters (Vol. 17, No. 17, pp 602-603, 1981) are indicative of the work of researchers in this field.

It is an object of the present invention to provide a biosensing device, for use in a liquid phase environment.

According to the present invention there is provided a biosensing device comprising a piezoelectric support having electrodes on the surface thereof characterised in that the support is a single crystal having the electrodes photolithographically deposited on the surface as elongate regions of metallisation transverse to the direction of propagation of a wave along the surface of the support, the elongate metallised regions having a width and spacing of the same order, and protein immobilised on the surface of the support.

Advantageously, the electrodes have a width of between 1 and 40µm preferably between 10 and 20µm.

The regions of metallisation forming the electrodes may be formed by the deposition of Chromium, on the surface of the support and the vapour deposition of gold onto the Chromium, and thereafter by etching to the desired pattern.

The electrodes may have a thickness of between 1000°A and 5µm, but preferably of the order of 2000°A.

Mass loading of the surface may be effected by the formation of a grating on the surface of the crystal, the grating being defined by the electrodes and/or by additional metallic fingers and/or by surface troughs interdigitated with the electrodes to form a grating.

A preferred embodiment of biosensor having an electrode width and spacing each of the order of 11µ on a crystal support having a resonant frequency greater than 100MHz, when immersed in a liquid phase test solution, produces a surface skimming bulk acoustic wave having a liquid penetration less than 500°A. As stated above, the provision of a metallised grating on the support surface, reduces the penetration of the support and the superjacent liquid by the surface skimming bulk acoustic wave.

The invention will be described further, by way of example, with reference to the accompanying drawings, in which:-

Fig.1 is a fragmentary diagrammatic view, to a greatly enlarged scale, of the surface region of a biosensing device according to the present invention;

Fig. 2 is a graph illustrating the relationship between operating frequency and penetration depth of surface skimming bulk acoustic wave motion into a supported protein (immobilised antibody) layer and;

Fig. 3 is a plan view of the uncoated surface of a biosensing device according to the invention.

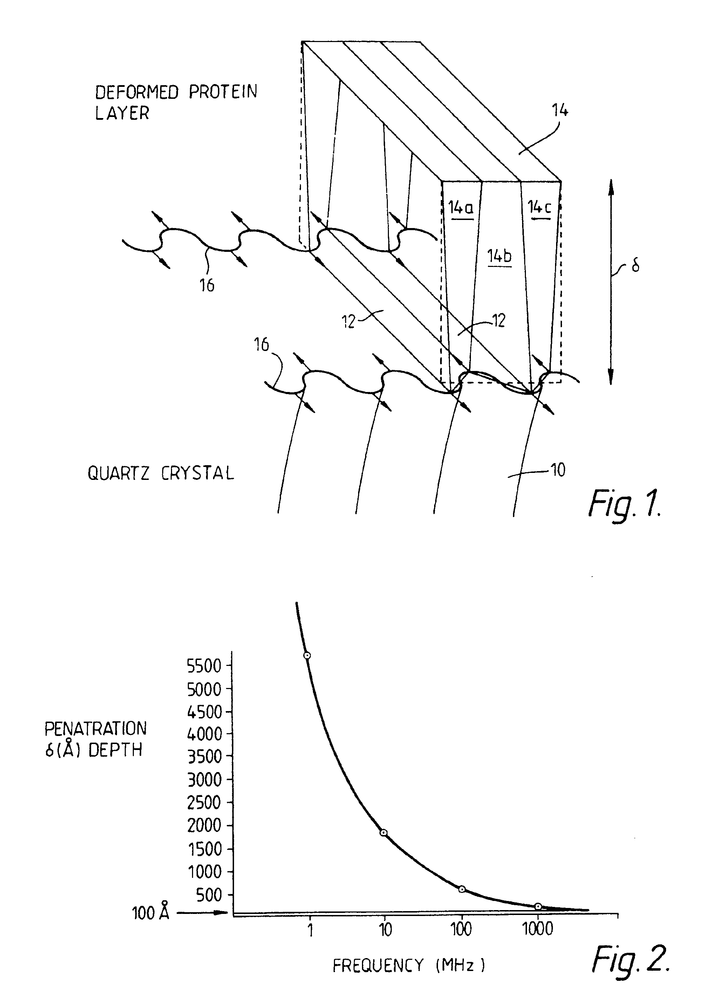

Referring to the drawings, a single quartz crystal forms a support 10 for the biosensing device. The crystal is cut on the ST axis and the surface is polished. Electrodes 12 (diagrammatically shown in Fig.1) are deposited on the surface parallel to the X axis of the crystal. The electrodes 12 are formed by photolithographic techniques (known per se) for example, deposition and subsequent etching, and comprise parallel strips having a thickness of approximately 200°A; a width of approximately 11μ and a spacing of approximately 20μ. The electrodes may be formed by the initial deposition of a layer of chromium. A layer of gold is then vapour deposited on to the surface of the chromium. The electrode pattern is then defined photolithographically using a resist and then etched.

Each chip 10 also includes a temperature sensing device 16 having leads 17 and contact pads 18. The final size of the biosensor is approximately 15mm x 10mm but it will be appreciated that a plurality of such devices are formed simultaneously on a wafer of the quartz, the individual chips being cut from the wafer at a later stage of manufacture.

The electrodes 12 are brought out by connections 13 to contact pads 15.

As shown in Fig. 1, a layer 14, containing a bonding agent and the desired antibody is coated on the surface of the support 10 over the electrodes 12. The layer 14 is arranged to be very thin, e.g. 500°A and preferably of the order of 100°A.

An A.C. signal of frequency, for example, 100 MHz, is applied to the contact pads and causes the production of motion in the crystal (diagrammatically illustrated in Fig.1) and the transmission of a surface skimming bulk acoustic wave 16 along the surface of the crystal transverse to the X axis (and to the electrodes). The layer 14 is deformed by the crystal motion as diagrammatically illustrated in Fig. 1 by the shape of the segments 14a, 14b and 14c of the surface protein layer 14. Immersion of the sensor in a liquid causes damping of the wave motion. However, the damping is very much reduced as compared with that of a conventional acoustic wave as shown in the comparative table I below which indicates attenuation in air and other media of a sensor according to the invention as compared to a known lithium niobate sensor operating at a lower frequency.

| Surface Contact Medium | (dB) Attenuation of SSBW[1] | (dB) Attenuation of SAW[2] |
|---|---|---|
| Air | negligible | negligible |
| Water | 0.1 | 20 |
| Glycerol | 1.0 | 28 |
| glue (after drying) | 33.0 | - |

1. 110 MHz ST Quartz - 20mm[2] coverage area.
2. 70MHz Lithium Niobate YZ - 2.4mm[2] coverage area.

Table 1.

The low attenuation of an SSBW produced by a sensor according to the present invention, upon immersion in a liquid, is due to the low penetration of the surface skimming bulk acoustic wave into the liquid. Fig. 2 is a graph illustrating this and showing the dependence of penetration on operating frequency. The sensitivity of the biosensor of the present invention can be maximised by operating at high frequencies (>100MHz) and utilising a thinly coated protein layer 14 on the surface of the support 10.

Binding of an antigen from the liquid to the antibody in the layer 14, is more readily detected, the thinner the layer 14 (the lower the mass of the layer 14). The consequent relative change in mass caused by binding of an antigen to the layer 14 is much higher. The change in mass occasions a change in velocity and hence of frequency of the wave. The change in frequency is detected and is indicative of the presence of an antigen.

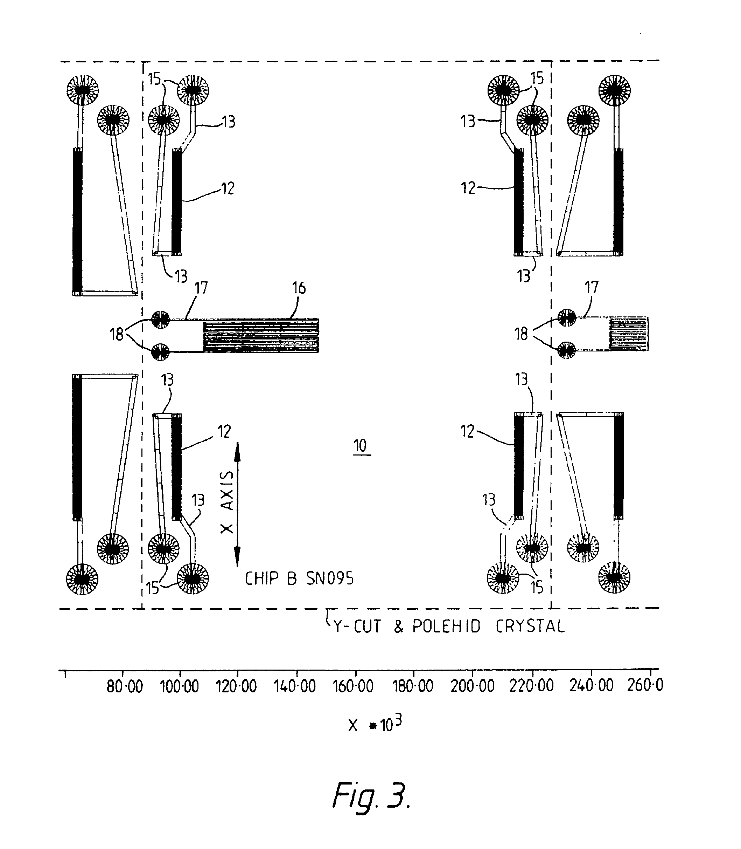

The invention is not confined to the precise details of the foregoing example and variations may be made thereto. For example, the penetration of the surface skimming bulk acoustic wave into the crystal support 10 can be reduced by increasing the effective mass loading of the surface of the support 10. The electrode structure 12 (Figs 1 and 3) can be increased in mass by narrowing the spacing and/or by the provision of additional interdigitated metallisation and/or by the provision of troughs in the surface of the crystal between the metallisation. The grating so formed may have a spacing of the order of $\lambda/2$ where $\lambda$ is the wavelength of the SSBW. Similarly, the thickness of the grating of the metallisation or of the metallisation and ridge height (where troughs are formed) is preferably less than $\lambda/100$. The use of a grating increases the surface propagation of the wave. The ideal sensor is one in which all the energy of the wave is confined to the protein layer 14, none being lost in the liquid or the crystal. Maximum sensitivity to antigen binding would thereby be achieved. The operating frequency of the device may be up to 500 MH2. The electrode structure 12 may be formed of Aluminium.

In use, a biosensor coated with an antibody containing layer 14 and constructed in accordance with the present invention is immersed in a liquid or has a spot of a liquid applied to the surface of the layer 14. If the liquid contains an antigen which will bind with the antibody of the layer 14, attenuation and consequent frequency change of the applied signal occurs and the antigen is positively detected without any masking occurring due to excessive attenuation of the wave energy by the liquid.

## Claims

1. A biosensing device comprising a piezoelectric support (10) having electrodes (12) on the surface thereof characterised in that the support (10) is a single crystal having the electrodes (12) photolithographically deposited on the surface thereof as elongate regions of metallisation transverse to the direction of wave propagation, the elongate metallised regions having their width and spacing of the same order, and a protein immobilised on the surface of the support (10).

2. A device as claimed in Claim 1, wherein the electrodes have a width of 10 to 20um, preferably 11um.

3. A device as claimed in Claim 1 or 2, wherein the elongate metallised regions comprise Aluminium or gold on chromium.

4. A device as claimed in Claim 3, wherein the elongate metallised regions comprise a layer of chromium on the crystal surface, a layer of gold vapour deposited on the surface, and a pattern formed by etching of the gold/chromium layer.

5. A device as claimed in Claim 2, 3 or 4, wherein the electrodes have a thickness between 1000°A and 5um.

6. A device as claimed in Claim 2, 3 or 4, wherein the thickness of the electrodes is 2 000°A.

7. A device as claimed in any one of the preceding claims, wherein the mass loading of the surface of the crystal is increased by the formation of a grating including of the elongate metallised regions.

8. A device as claimed in Calim 7, further including additional metallic fingers interdigitated between the elongate regions of metallisation.

9. A device as claimed in Claim 7 or 8, further including troughs in the surface of the crystal between the elongate regions of metallisation.

10. A method of maunfacturing a biosensing device comprising the steps of cutting a crystal to form a Y-cut single crystal support (10), polishing the surface thereof, depositing a layer of a metal on the surface, applying a resist and photolithographically forming a plurality of elongate regions in the metal layer to provide a pattern of electrodes (12) substantially parallel to the X axis of the crystal and forming, on the surface of the crystal over the electrodes, a layer (14) including a protein.

DEFORMED PROTEIN LAYER

QUARTZ CRYSTAL

Fig. 1.

PENATRATION
δ(Å) DEPTH

100 Å

FREQUENCY (MHz)

Fig. 2.

15

13

12

13

17 16

18

10

X AXIS

CHIP B SN095

13

12

13

15

15

13

13

12

18 17

13

13

12

13

15

Y-CUT & POLEHID CRYSTAL

80·00 100·00 120·00 140·00 160·00 180·00 200·00 220·00 240·00 260·0

$X *10^3$

*Fig. 3.*

7

**European Patent Office**

# EUROPEAN SEARCH REPORT

Application Number

EP   91 30 3314

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-361729 (HEWLETT-PACKARD) * column 1, line 6 - column 8, line 54; figures 1, 5 * | 1, 2, 5, 7, 8 | G01N27/00 G01N29/00 |
| Y | * idem * | 3, 4, 6, 9, 10 | |
| X | US-A-4735906 (G.J BASTIAANS ET AL) * column 3, line 26 - column 4, line 54 * * column 6, line 36 - column 7, line 6 * * figures 1, 2 * | 1 | |
| A | | 10 | |
| Y | ANALYTICAL CHEMISTRY. vol. 56, no. 8, July 1984, COLUMBUS US pages 1411 - 1416; A. SNOW ET AL: "Poly<ethylene maleate>-cyclopentadiene: a model reactive polymer-vapor system for evaluation of a SAW microsensor" * the whole document * | 3, 4, 6, 10 | |
| A | * idem * | 1, 2 | |
| Y | WO-A-8702134 (NEDERLANDSE CENTRALE ORGANISATIE VOOR TOEGEPAST NATUURWETENSCHAPPELIJK ONDERZOEK) * page 1, line 1 - page 5, line 36; figure 1 * | 9 | TECHNICAL FIELDS SEARCHED (Int. Cl.5) |
| A | * idem * | 1, 7 | G01N |
| A | PROCEEDINGS OF IEEE 1988 ULTRASONICS SYMPOSIUM. vol. 1, ed. B.R. McAVOY, 1989, NEW YORK US pages 607 - 611; S.J. MARTIN ET AL: "Sensing in liquids with SH plate mode devices" * the whole document * | 1, 3, 5, 10 | |
| D,A | JOURNAL OF THE ACOUSTICAL SOCIETY OF AMERICA. vol. 84, no. 3, September 1988, NEW YORK US pages 978 - 984; F. JOSSE et AL: "Analysis of shear horizontal surface waves at the boundary between a piezoelectric crystal and a viscous fluid medium" * the whole document * | 1 | |

The present search report has been drawn up for all claims

| Place of search | Date of completion of the search | Examiner |
|---|---|---|
| BERLIN | 05 SEPTEMBER 1991 | JOHNSON, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

8